# EUROPEAN PATENT APPLICATION

(11) **EP 3 546 938 A1**
(43) Date of publication of application: **02.10.2019**
(21) Application number: 18305375.0
(22) Date of filing: 30.03.2018
(51) Int. Cl.: G01N 33/53, G01N 33/68

(54) **METABOLIC SIGNATURE AND USE THEREOF FOR THE DIAGNOSIS OF GLAUCOMA**

(71) Applicant: Université d'Angers, 49000 Angers (FR); Centre Hospitalier Universitaire d'Angers, 49100 Angers (FR); Centre National de la Recherche Scientifique, 75016 Paris (FR); INSERM (Institut National de la Santé et de la Recherche Médicale), 75654 Paris Cedex 13 (FR)
(72) Inventor: REYNIER, Pascal, 49460 Montreuil-Juigné (FR); LERUEZ, Stéphanie, 49100 Angers (FR); PROCACCIO, Vincent, 49240 Avrillé (FR); CHAO DE LA BARCA, Juan Manuel, 49000 Angers (FR); LENAERS, Guy, 49240 Avrillé (FR); BONNEAU, Dominique, 49100 Angers (FR); GOHIER, Philippe, 49100 Angers (FR)
(74) Representative: Icosa

(57) **Abstract**

The present invention relates to a metabolic signature comprising at least four metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine, wherein said metabolic signature is indicative of glaucoma in a subject. The present invention also relates to a method for diagnosing glaucoma in a subject and/or for identifying a subject as being at risk for glaucoma. The present invention also relates to a method for diagnosing glaucoma in a subject and/or for identifying a subject as being at risk for glaucoma. The present invention also relates to a kit for implementing the method of the invention.

## Description

### FIELD OF INVENTION

The present invention relates to a metabolic signature of glaucoma, comprising metabolites selected from biogenic amines, amino acids, fatty acid esters, carbohydrates and/or glycerophospholipids.

The present invention also relates to methods using the metabolic signature of the invention, for diagnosing a subject with or identifying a subject as being at risk of developing glaucoma.

The present invention also relates to kits for implementing the methods of the invention.

### BACKGROUND OF INVENTION

Glaucoma is a group of chronic optic neuropathies characterized by the progressive loss of retinal ganglion cells, the presence of cupped optic neuropathy, irreversible visual impairment, loss of the visual field and potential blindness (Choplin & Traverso (Eds.), 2014. Atlas of glaucoma (3rd ed.). Boca Raton, FL: CRC Press Inc.). This disease, affecting almost 65 million people worldwide, and increasing to nearly 80 million by 2020, is the most frequent cause of irreversible blindness (Quigley & Broman, 2006. Br J Ophthalmol. 90(3):262-7; Stevens et al., 2013. Ophthalmology. 120(12):2377-84; Tham et al., 2014. Ophthalmology. 121(11):2081-90).

Glaucoma can be divided into 3 various types: open-angle glaucoma (OAG), angle-closure glaucoma (ACG) and congenital glaucoma (CG); and into primary or secondary subtypes.

Primary open-angle glaucoma (POAG), the most common form of glaucoma, is defined by the presence of a glaucomatous optic neuropathy without an identifiable cause. The pathogenesis of POAG is multifactorial and remains to be elucidated. The principal risk factors are old age and elevated intraocular pressure, with systemic, environmental and genetic factors, high myopia, as well as gender and ethnic origins also contributing to the disease (Jonas et al., 2017. Lancet. 390(10108):2183-2193).

Early treatment of POAG consists in lowering the intraocular pressure (IOP), to inhibit progression of vision loss. However, this treatment is not always totally successful and rarely reverses established damages. Therefore, to achieve satisfactory therapeutic results, early detection is of major importance.

Diagnosing and following the clinical course of a subject with glaucoma currently relies on ophthalmological criteria (such as, for example, elevated IOP, loss of visual acuity, smaller visual field, or presence of optic nerve damage with progressive optic disc cupping) that are only apparent once the disease has progressed into irreversible loss of the visual field and damage to the optic nerve.

Lately, studies have been carried out to try to identify causative glaucoma-associated genes, with the aim of developing risk prediction and early detection tools. Association and linkage-based glaucoma genetic studies have identified loci contributing to susceptibility to glaucoma, or to phenotypic features associated with risk of glaucoma (Fan et al., 2011. Invest Ophthalmol Vis Sci. 52(3):1788-92). Genes including myocilin, CYP1B1 and optineurin lead to early onset, juvenile or congenital glaucoma, and in some cases, to adult-onset POAG. Susceptibility alleles in the LOXL1 gene confer risk of exfoliative glaucoma (PEG), which is a secondary subtype OAG (Thorleifsson et al., 2007. Science. 317(5843):1397-400). Two loci, CDKN2B-AS1 and SIX1/SIX6, were also identified as strongly associates with optic nerve degeneration (Wiggs et al., 2012. PLoS Genet. 8(4):e1002654). Others have reported an association of the CDKN2B-AS1, CAV1/CAV2, TMCO1 and GAS7 loci in POAG (Thorleifsson et al., 2010. Nat Genet. 42(10):906-9; Burdon et al., 2011. Nat Genet. 43(6):574-8). However, taken individually, these references explain only a limited portion of cases of POAG.

International patent application WO2015171457 discloses a method for predicting onset, progression, recurrence and/or severity of POAG, based on the analysis of allelic information or expression levels of a collection of over 250 molecular signature biomarkers.

However, such genetic signature is not fully illustrative of the physiological state of a subject. In comparison to genetic or proteomic signatures, metabolic signatures can reveal the direct and indirect fingerprints of a disease. Indeed, metabolomics produce profiles that are closer to the phenotype of the disease, integrating several other factors in addition to the sole genetic factors. These include the effect of age, the way of life, nutrition, physical activities, medication and so on. The human metabolome is estimated to contain more than 80000 metabolites (Human Metabolome Database, http://www.hmdb.ca; Wishart et al., 2018. Nucleic Acids Res. 46(D1):D608-D617).

To date, three metabolomic studies have been carried out on subjects affected with glaucoma (Mayordomo-Febrer et al., 2015. Exp Eye Res. 131:84-92; Rong et al., 2017. Biomed Chromatogr. 31(9**)**; Burgess et al., 2015. Invest Ophthalmol Vis Sci. 56(8):5020-8), but only one focused on subjects affected with POAG (Burgess et al., 2015. Invest Ophthalmol Vis Sci. 56(8):5020-8).

Burgess *et al.* compares, in an untargeted metabolomic approach, the plasma metabolome of individuals with POAG with that of controls, using high resolution mass spectrometry. This study revealed 41 discriminant metabolites including palmitoylcarnitine, hydroxyergocalciferol, sphingolipids, vitamin D-related molecules and terpenes, indicating perceptible alterations of the blood during fatty acid metabolism, steroid biosynthesis, and sphingolipid metabolism.

However, such untargeted approach is not without bias. Indeed, in untargeted approaches, samples are analyzed without authentic standards, which compromises the quantitative accuracy and confidence in metabolite identification.

Therefore, there remains a need for a robust and clinically-relevant metabolic signature of POAG, that would allow an earlier diagnostic and a better stratification of patients on pathophysiological criteria.

Here, the Inventors have established such metabolic signature using a targeted metabolomic approach. Surprisingly, they have been able to identify 18 discriminating plasmatic metabolites, whose measure allows an accurate diagnosis of POAG patients.

### SUMMARY

The invention relates to a metabolic signature comprising at least four, preferably at least twelve metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine, wherein said metabolic signature is indicative of glaucoma in a subject.

In one embodiment, the amount, level or concentration of at least 1 metabolite, preferably at least 2 metabolites selected from the group comprising histamine, octadecadienoylcarnitine, octadecenoylcarnitine, spermidine and spermine is lower, and the amount, level or concentration of at least 1 metabolite, preferably at least 2 metabolites selected from the group comprising arginine, butyrylcarnitine, decenoylcarnitine, dodecenoylcarnitine, hexoses, methionine, methionine sulfoxide, PC aa 34:2, PC aa 34:4, PC aa 36:4, PC ae 40:1, propionylcarnitine and tyrosine is higher, with respect to the amount, level or concentration of the same metabolite in a reference population.

In one embodiment the reference population comprises at least 10 subjects who are not affected and/or who have not been diagnosed with glaucoma.

The invention also relates to a method for diagnosing glaucoma in a subject and/or for identifying a subject as being at risk for glaucoma, comprising the steps of:
a. measuring in a sample previously obtained from the subject the amount, level or concentration of at least four metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine, thereby obtaining a personalized metabolic signature,
b. comparing the personalized metabolic signature obtained in step b) with the metabolic signature as described hereinabove, and
c. diagnosing the subject as being affected or as being at risk of being affected with glaucoma when the comparison of step c) reveals that the personalized metabolic signature provided in step b) is substantially similar to the metabolic signature as described hereinabove.

The invention also relates to a method for diagnosing glaucoma in a subject and/or for identifying a subject as being at risk for glaucoma, comprising the steps of:
a. measuring in a sample previously obtained from the subject the amount, level or concentration of at least four metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine,
b. comparing the amount, level or concentration of the at least four, preferably at least twelve, more preferably eighteen metabolites measured in step b) with the amount, level or concentration of the same at least four, preferably at least twelve, more preferably eighteen metabolites in a population of substantially healthy subjects, and
c. diagnosing the subject as being affected or as being at risk of being affected with glaucoma when
   - the amount, level or concentration of at least 2 metabolites selected from the group comprising histamine, octadecadienoylcarnitine, octadecenoylcarnitine, spermidine and spermine measured in step b) is lower than the amount, level or concentration of the same metabolite in a reference population of substantially healthy subjects, and
   - the amount, level or concentration of at least 2 metabolites selected from the group comprising arginine, butyrylcarnitine, decenoylcarnitine, dodecenoylcarnitine, hexoses, methionine, methionine sulfoxide, PC aa 34:2, PC aa 34:4, PC aa 36:4, PC ae 40:1, propionylcarnitine and tyrosine measured in step b) is higher than the amount, level or concentration of the same metabolite in a reference population of substantially healthy subjects.

In one embodiment, the step a) of the methods of the invention comprises measuring the amount, level or concentration of at least twelve metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine.

In one embodiment, the step a) of the methods of the invention comprises measuring the amount, level or concentration of eighteen metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine.

In one embodiment, the amount, level or concentration of metabolites is measured by flow injection analysis coupled with tandem mass spectrometry (FIA-MS/MS) or by liquid chromatography coupled with mass spectrometry (LC-MS).

In one embodiment, the glaucoma is open-angle glaucoma, preferably glaucoma is primary open-angle glaucoma.

In one embodiment, the sample is a blood sample, preferably a plasma sample.

The invention also relates to a kit for implementing the method of the invention, wherein said kit comprises:
- means for determining the amount, level or concentration of at least one metabolite selected from the group comprising arginine, butyrylcarnitine, decenoylcarnitine, dodecenoylcarnitine, hexoses, histamine, methionine, methionine sulfoxide, octadecadienoylcarnitine, octadecenoylcarnitine, PC aa 34:2, PC aa 34:4, PC aa 36:4, PC ae 40:1, propionylcarnitine, spermidine, spermine and tyrosine,
- internal standards and/or control, and
- instructions for use.

In one embodiment, the internal standards and/or control are selected from:
- stable isotope-labelled arginine, stable isotope-labelled butyrylcarnitine, stable isotope-labelled decenoylcarnitine, stable isotope-labelled dodecenoylcarnitine, stable isotope-labelled hexoses, stable isotope-labelled histamine, stable isotope-labelled methionine, stable isotope-labelled methionine sulfoxide, stable isotope-labelled octadecadienoylcarnitine, stable isotope-labelled octadecenoylcarnitine, stable isotope-labelled PC aa 34:2, stable isotope-labelled PC aa 34:4, stable isotope-labelled PC aa 36:4, stable isotope-labelled PC ae 40:1, stable isotope-labelled propionylcarnitine, stable isotope-labelled spermidine, stable isotope-labelled spermine, stable isotope-labelled tyrosine, and/or
- stable isotope-labelled isobaric metabolites having the same molecular mass but different chemical formula as arginine, butyrylcarnitine, decenoylcarnitine, dodecenoylcarnitine, hexoses, histamine, methionine, methionine sulfoxide, octadecadienoylcarnitine, octadecenoylcarnitine, PC aa 34:2, PC aa 34:4, PC aa 36:4, PC ae 40:1, propionylcarnitine, spermidine, spermine or tyrosine.

### DEFINITIONS

In the present invention, the following terms have the following meanings:
The terms **"amount", "level"** or **"concentration"** are used interchangeably and refer to the measured quantity of a metabolite in a sample from a subject.

The term **"diagnosis",** as used herein, refers to medical diagnosis, the process of determining which disease explain the symptoms of a subject.

The term **"glaucoma",** as used herein, refers to a group of disorders characterized by a progressive optic neuropathy resulting in a characteristic appearance of the optic disc and a specific pattern of irreversible visual field defects - eventually leading to vision loss and blindness - that are associated frequently but not invariably with raised intraocular pressure (IOP). According to the European Glaucoma Society, glaucoma can be devised into several types (2017. Br J Ophthalmol. 101(5):73-127):
**1.** Open-angle glaucoma (AOG): OAG is a chronic progressive optic neuropathy with characteristic morphological changes at the optic nerve head and retinal nerve fiber layer in the absence of other ocular disease or congenital anomalies. Progressive retinal ganglion cell death and visual field loss are associated with these changes.
   **1.1.** Primary OAG (PAOG): POAG refers to OAG that is not caused by another eye or medical condition.
      **1.1.1.** High-tension glaucoma (HTG): HTG is a common form of POAG in which there is a measured elevation of the intraocular pressure (IOP), *i.e.,* IOP > about 21 mmHg.
      **1.1.2.** Normal-tension glaucoma (NTG): NTG is a common form of POAG in which there is no measured elevation of the intraocular pressure (IOP), *i.e*., IOP < about 21 mmHg.
      **1.1.3.** Primary juvenile OAG.
      **1.1.4.** POAG suspect.
      **1.1.5.** Ocular hypertension.
   **1.2.** Secondary Open-Angle Glaucoma (SAOG): SOAG refers to OAG that is caused by another eye or medical condition.
      **1.2.1.** SAOG caused by ocular disease, including, but not limited to, pseudoexfoliative glaucoma, pigmentary glaucoma, lens-induced AOG, glaucoma associated with intraocular haemorrhage, uveitic glaucoma, neovascular glaucoma, glaucoma due to intraocular tumor, glaucoma associated with retinal detachment.
      **1.2.2.** SAOG due to ocular trauma.
      **1.2.3.** Iatrogenic SOAG, including, but not limited to, glaucoma due to corticosteroid treatment and glaucoma due to ocular surgery and laser.
      **1.2.4.** SOAG caused by extrabulbar disease, including, but not limited to, glaucoma caused by increased episcleral venous pressure.
**2.** Angle-Closure Glaucoma (ACG)
   **2.1.** Primary ACG (PACG): PACG refers to ACG that is not caused by another eye or medical condition.
      **2.1.1.** Primary angle-closure suspect (PACS), also known as occludable angle glaucoma.
      **2.1.2.** Acute angle-closure (AAC).
         **2.1.2.1.** AAC with pupillary block mechanism.
         **2.1.2.2.** AAC with plateau iris configuration.
      **2.1.3.** Intermittent angle-closure (IAC).
      **2.1.4.** Chronic angle-closure glaucoma (CACG).
      **2.1.5.** Status post-acute angle-closure attack.
   **2.2.** Secondary ACG (SACG): SACG refers to ACG that is not caused by another eye or medical condition.
      **2.2.1.** SACG with pupillary block.
      **2.2.2.** SACG with anterior pulling mechanism without pupillary block, including, but not limited to, neovascular glaucoma, iridocorneal endothelial syndrome, posterior polymorphous dystrophy, epithelial and fibrous ingrowth after anterior segment surgery or penetrating trauma, inflammatory membrane, peripheral anterior synechiae after laser trabeculoplasty and endothelial membrane covering the trabecular meshwork late after laser trabeculoplasty, and aniridia.
      **2.2.3.** SACG with posterior pulling mechanism without pupillary block, including, but not limited to, aqueous misdirection (also known as cilio-lenticular block, ciliary block or malignant glaucoma), iris and ciliary body cysts, intraocular tumors, silicon oil or other tamponading fluids or gas implanted in the vitreous cavity, uveal effusion, retinopathy of prematurity and congenital anomalies.
**3.** Primary Congenital Glaucoma (PCG).

The term **"metabolite",** as used herein, refers to a small molecule that is an intermediate or a product of life-sustaining chemical transformation within the cells of an organism.

The term **"patient population",** as used herein, refers to a group of subjects with shared factors, influences or condition. In one embodiment, a patient population comprising at least 1, preferably at least 5, at least 10, at least 15, at least 20, at least 25, at least 30, at least 35, at least 40, at least 45, at least 50, at least 75, at least 100 or more subjects diagnosed with glaucoma, *i.e.,* of known glaucoma outcome.

The term **"patient signature",** as used herein, refers to a standard pattern of metabolites' amounts, levels or concentrations measured in a "patient population". In one embodiment, the metabolites' amounts, levels or concentrations are absolute metabolite concentrations. In one embodiment, the metabolite amounts, levels or concentrations are relative metabolite concentrations.

The term **"reference population",** as used herein, refers to a group of subjects with shared factors, influences or condition. In one embodiment, a reference population comprising at least 1, preferably at least 5, at least 10, at least 15, at least 20, at least 25, at least 30, at least 35, at least 40, at least 45, at least 50, at least 75, at least 100 or more substantially healthy subjects, *i.e.,* subject who are not affected and/or who have not been diagnosed with glaucoma.

The term **"reference signature",** as used herein, refers to a standard pattern of metabolites' amounts, levels or concentrations measured in a "reference population". In one embodiment, the metabolites' amounts, levels or concentrations are absolute metabolite concentrations. In one embodiment, the metabolite amounts, levels or concentrations are relative metabolite concentrations.

The term **"sample",** as used herein, refers to any biological material obtained via suitable methods known to the person skilled in the art from a subject. The sample may be collected in a clinically acceptable manner, *e.g.,* in a way that cells, nucleic acids (such as DNA and RNA), proteins and/or metabolites are preserved. A **"sample"** may include body tissue and/or bodily fluids. Examples of bodily fluids include, but are not limited to, blood, plasma, serum, urine, amniotic fluid, cerebrospinal fluid, saliva and aqueous humor.

The term **"subject",** as used herein, refers to an animal, preferably a mammal, more preferably a human. In one embodiment, the subject is a patient, *i.e.,* a recipient of health care services.

### DETAILED DESCRIPTION

The present invention relates to a metabolic signature of glaucoma, wherein said metabolic signature comprises metabolites whose amount, level or concentration are different between subjects affected by glaucoma and substantially healthy subjects.

In one embodiment, the metabolic signature of glaucoma of the present invention is a metabolic signature of open-angle glaucoma (OAG). OAG includes, but is not limited to, primary OAG (POAG) and secondary OAG (SOAG).

In one embodiment, the metabolic signature of glaucoma of the present invention is a metabolic signature of POAG. POAG includes, but is not limited to, high-tension glaucoma (HTG), normal-tension glaucoma (NTG), primary juvenile OAG (PJOAG), POAG suspect and ocular hypertension.

In one embodiment, the metabolic signature of glaucoma of the present invention comprises or consists of at least one metabolite. In one embodiment, the metabolic signature of glaucoma of the present invention comprises or consists of at least 4 metabolites. In one embodiment, the metabolic signature of glaucoma of the present invention comprises or consists of at least 7 metabolites. In one embodiment, the metabolic signature of glaucoma of the present invention comprises or consists of at least 12 metabolites. In one embodiment, the metabolic signature of glaucoma of the present invention comprises or consists of at least 13 metabolites. In one embodiment, the metabolic signature of glaucoma of the present invention comprises or consists of at least 18 metabolites.

In one embodiment, the metabolic signature of glaucoma of the present invention comprises or consists of at least one metabolite selected from the group of biogenic amines.

Examples of biogenic amines include, but are not limited to, spermidine, spermine, histamine, serotonin, epinephrine, dopamine, norepinephrine, phenethylamine, N-methylphenethylamine, phenylethanolamine, m-tyramine, p-tyramine, 3-methoxytyramine, *N*-methyltyramine, m-octopamine, p-octopamine, synephrine, thyronamine, 3-iodothyronamine, tryptamine, *N*-methyltryptamine, *N,N-*dimethyltryptamine, trimethylamine, trimethylamine *N*-oxide, agmatine, adaverine, cadaverine and putrescine.

In a preferred embodiment, the at least one metabolite selected from the group of biogenic amines is selected from spermidine, spermine and histamine.

In one embodiment, the metabolic signature of glaucoma of the present invention comprises or consists of at least one metabolite selected from the group of amino acids.

Examples of amino acids include, but are not limited to, methionine, methionine sulfoxide, arginine, tyrosine, alanine, asparagine, aspartic acid, cysteine, glutamic acid, glutamine, glycine, histidine, isoleucine, leucine, lysine, phenylalanine, proline, serine, threonine, tryptophan, valine, selenocysteine and pyrrolysine.

In a preferred embodiment, the at least one metabolite selected from the group of amino acids is selected from methionine, methionine sulfoxide, arginine and tyrosine.

In one embodiment, the metabolic signature of glaucoma of the present invention comprises or consists of at least one metabolite selected from the group of fatty acid esters.

Examples of fatty acid esters include, but are not limited to, carninite (C0), acylcarnitine (C2), propionylcarnitine (C3), butyrylcarnitine (C4), isobutyrylcarnitine (C4), 3-OH-butyrylcarnitine (C4-OH), succinylcarnitine (C4DC), pivaloylcarnitine (C5), 2-methylbutyrylcarnitine (C5), valerylcarnitine (C5), isovalerylcarnitine (C5), 3-OH-isovalerylcarnitine (C5-OH), tiglylcarnitine (C5:1), 3-methylcrotonylcarnitine (C5:1), glutarylcarnitine (C5DC), hexanoylcarnitine (C6), octanoylcarnitine (C8), decanoylcarnitine (C10), decenoylcarnitine (C10:1), dodecanoylcarnitine (C12), dodecenoylcarnitine (C12:1), tetradecanoylcarnitine (C14), 3-OH-tetradecenoylcarnitine (C14:OH), tetradecenoylcarnitine (C14:1), 3-OH-tetradecanoylcarnitine (C14:1-OH), tetradecanoylcarnitine (C14:2), hexadecanoylcarnitine (C16), 3-OH-hexadecanoylcarnitine (C16-OH), 3-OH-hexadecanoylcarnitine (C16:1-OH), hexadecadicarboxylcarnitine (C16DC), octadecanoylcarnitine (C18), 3-OH-octadecenoylcarnitine (C18:OH), octadecenoylcarnitine (C18:1), 3-OH-octadecadienoylcarnitine (C18:1-OH), octadecadienoylcarnitine (C18:2), glycerol monostearate, glycerol monopalmitate, glycerol monomyristate, glycerol monocaprate, glycerol monodecanoate, glycerol monolaurate, glycerol monolinoleate, glycerol monooleate, diglycerides and triglycerides.

In a preferred embodiment, the at least one metabolite selected from the group of fatty acid esters is selected from propionylcarnitine (C3), butyrylcarnitine (C4), decenoylcarnitine (C10:1), dodecenoylcarnitine (C12:1), octadecenoylcarnitine (C18:1) and octadecadienoylcarnitine (C18:2).

In one embodiment, the metabolic signature of glaucoma of the present invention comprises or consists of at least one metabolite selected from the group of carbohydrates. Carbohydrates include, but are not limited to, monosaccharides, disaccharides, oligosaccharides and polysaccharides.

Examples of monosaccharides include, but are not limited to, dioses (such as glycolaldehyde), trioses (such as dihydroxyacetone and glyceraldehyde), tetroses (such as erythrulose, erythrose and threose), pentoses (such as ribulose, xylulose, arabinose, lyxose, ribose and xylose), hexoses (such as allose, altrose, galactose, glucose, gulose, idose, mannose, talose, fructose, psicose, sorbose, tagatose, fucose, fuculose and rhamnose), heptoses (such as mannoheptulose and sedoheptulose), octoses and nonoses.

Examples of disaccharides include, but are not limited to, cellobiose, gentiobiose, inulobiose, isomaltose, isomaltulose, kojibiose, lactose, lactulose, laminaribiose, leucrose, maltose, maltulose, melibiose, nigerose, robinose, rutinose, saccharose, sophorose, trehalose, trehalulose and turanose.

Examples of oligosaccharides include, but are not limited to, erlose, fucosyllactose, gentianose, inulotriose, 1-kestose, 6-kestose, maltotriose, mannotriose, melezitose, neokestose, panose, raffinose, rhamninose, cyclodextrine, stachyose and verbascose.

Examples of polysaccharides include, but are not limited to, beta-glucan, lentinan, sizofiran, zymosan, cellulose, chitin, chitosan, dextran, fructan, inulin, galactan, glucan, glycogen, hemicellulose, levan β2→6, lignin, mannan, pectin, starch, amylopectin, amylose and xanthan.

In a preferred embodiment, the at least one metabolite selected from the group of carbohydrates is selected from hexoses.

In one embodiment, the metabolic signature of glaucoma of the present invention comprises or consists of at least one metabolite selected from the group of glycerophospholipids.

Examples of glycerophospholipids include, but are not limited to, PC aa C24:0, PC aa C26:0, PC aa C28:1, PC aa C30:0, PC aa C30:2, PC aa C32:0, PC aa C32:1, PC aa C32:2, PC aa C32:3, PC aa C34:1, PC aa C34:2, PC aa C34:3, PC aa C34:4, PC aa C36:0, PC aa C36:1, PC aa C36:2, PC aa C36:3, PC aa C36:4, PC aa C36:5, PC aa C36:6, PC aa C38:0, PC aa C38:1, PC aa C38:3, PC aa C38:4, PC aa C38:5, PC aa C38:6, PC aa C40:1, PC aa C40:2, PC aa C40:3, PC aa C40:4, PC aa C40:5, PC aa C40:6, PC aa C42:0, PC aa C42:1, PC aa C42:2, PC aa C42:4, PC aa C42:5, PC aa C42:6, PC ae C30:0, PC ae C30:1, PC ae C30:2, PC ae C32:1, PC ae C32:2, PC ae C34:0, PC ae C34:1, PC ae C34:2, PC ae C34:3, PC ae C36:0, PC ae C36:1, PC ae C36:2, PC ae C36:3, PC ae C36:4, PC ae C36:5, PC ae C38:0, PC ae C38:1, PC ae C38:2, PC ae C38:3, PC ae C38:4, PC ae C38:5, PC ae C38:6, PC ae C40:0, PC ae C40:1, PC ae C40:2, PC ae C40:3, PC ae C40:4, PC ae C40:5, PC ae C40:6, PC ae C42:0, PC ae C42:1, PC ae C42:2, PC ae C42:3, PC ae C42:4, PC ae C42:5, PC ae C44:3, PC ae C44:4, PC ae C44:5, PC ae C44:6, lysoPC a C14:0, lysoPC a C16:0, lysoPC a C16:1, lysoPC a C17:0, lysoPC a C18:0, lysoPC a C18:1, lysoPC a C18:2, lysoPC a C20:3, lysoPC a C20:4, lysoPC a C24:0, lysoPC a C26:0, lysoPC a C26:1, lysoPC a C28:0, lysoPC a C28:1, SM (OH) C14:1, SM C16:0, SM C16:1, SM (OH) C16:1, SM C18:0, SM C18:1, SM C20:2, SM C22:3, SM (OH) C22:1, SM (OH) C22:2, SM C24:0, SM C24:1, SM (OH) C24:1, SM C26:0 and SM C26:1,
wherein "PC aa" refers to phosphatidylcholine diacyl,
wherein "PC ae" refers to phosphatidylcholine acyl-alkyl,
wherein "lysoPC a" refers to lysophosphatidylcholine acyl,
wherein "SM" refers to sphingomyelin,
wherein "SM (OH)" refers to hydroxysphingomyelin, and
wherein "CX:Y" represents the sum of the lengths of two alkyl chains (X) and the sum of the unsaturation in both chains (Y).

In a preferred embodiment, the at least one metabolite selected from the group of glycerophospholipids is selected from PC aa C34:2, PC aa C34:4, PC aa C36:4 and PC ae C40:1.

In one embodiment, the metabolic signature of glaucoma of the present invention comprises or consists of at least one metabolite selected from the group of biogenic amines, at least one metabolite selected from the group of amino acids, at least one metabolite selected from the group of fatty acid esters, at least one metabolite selected from the group of carbohydrates and/or at least one metabolite selected from the group of glycerophospholipids.

In one embodiment, the metabolic signature of glaucoma of the present invention comprises or consists of at least 4 metabolites selected from the group comprising or consisting of biogenic amines, amino acids, fatty acid esters, carbohydrates and glycerophospholipids.

In one embodiment, the metabolic signature of glaucoma of the present invention comprises or consists of at least 4 metabolites selected from the group comprising or consisting of decenoylcarnitine (C10:1), octadecadienoylcarnitine (C18:2), PC ae 40:1 and spermidine.

In one embodiment, the metabolic signature of glaucoma of the present invention comprises or consists of at least 7 metabolites selected from the group comprising or consisting of biogenic amines, amino acids, fatty acid esters, carbohydrates and glycerophospholipids.

In one embodiment, the metabolic signature of glaucoma of the present invention comprises or consists of at least 7 metabolites selected from the group comprising or consisting of hexoses, methionine, octadecadienoylcarnitine (C18:2), PC ae 40:1, spermidine, spermine and tyrosine.

In one embodiment, the metabolic signature of glaucoma of the present invention comprises or consists of at least 12 metabolites selected from the group comprising or consisting of biogenic amines, amino acids, fatty acid esters, carbohydrates and glycerophospholipids.

In one embodiment, the metabolic signature of glaucoma of the present invention comprises or consists of at least 12 metabolites selected from the group comprising or consisting of arginine, butyrylcarnitine (C4), decenoylcarnitine (C10:1), dodecenoylcarnitine (C12:1), hexoses, histamine, methionine, octadecadienoylcarnitine (C18:2), PC ae 40:1, spermidine, spermine and tyrosine.

In one embodiment, the metabolic signature of glaucoma of the present invention comprises or consists of at least 13 metabolites selected from the group comprising or consisting of biogenic amines, amino acids, fatty acid esters, carbohydrates and glycerophospholipids.

In one embodiment, the metabolic signature of glaucoma of the present invention comprises or consists of at least 13 metabolites selected from the group comprising or consisting of hexoses, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine.

In one embodiment, the metabolic signature of glaucoma of the present invention comprises or consists of at least 18 metabolites selected from the group comprising or consisting of biogenic amines, amino acids, fatty acid esters, carbohydrates and glycerophospholipids.

In one embodiment, the metabolic signature of glaucoma of the present invention comprises or consists of at least 18 metabolites selected from the group comprising or consisting of arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine.

The amount, level or concentration of a metabolite is determined by physical methods, chemical methods or a combination thereof, which are well-known in the art. Such methods include, but are not limited to, mass spectrometry, liquid chromatography, Raman spectroscopy, emission spectroscopy, absorbance spectroscopy, circular dichroism spectroscopy, nuclear magnetic resonance (NMR), thin-layer chromatography, affinity chromatography, gas chromatography, size-exclusion chromatography, and combinations thereof.

In one embodiment, the amount, level or concentration of a metabolite is determined by flow injection analysis coupled with tandem mass spectrometry (FIA-MS/MS). Such approach is described in the example section of the present application.

In one embodiment, the amount, level or concentration of a metabolite is determined by liquid chromatography coupled with mass spectrometry (LC-MS). Such approach is described in the example section of the present application.

For quantification of metabolite concentration, NMR and MS-based are the most widely used techniques. NMR is the only detection technique which does not rely on separation of the metabolites, and the sample can thus be recovered for further analyses, *i.e*., all kinds of metabolites can be measured simultaneously. The main advantages of NMR are high analytical reproducibility and simplicity of sample preparation. Practically however, it is relatively insensitive compared to MS-based techniques, and requires much larger (from 10 to 100 times larger) sample volumes than MS.

MS is used to identify and to quantify metabolites after separation by liquid chromatography (LC) or gas chromatography (GC). MS is sensitive and can be very specific. MS-based technologies can further be classified as non-targeted approaches (*e.g.,* GC-MS and LC-MS) and targeted metabolomics, which is based on pre-selected multiple reaction monitoring (MRM) pairs and isotope-labeled internal standards, with many of their methods being based on rapid direct flow injection (FIA), however with capacity to carry out multiple GC-MS and LC-MS protocols. FIA-MS is especially adapted to high-throughput assays when large populations or time-series with many data points are investigated.

In one embodiment, if the amount, level or concentration of a metabolite is determined by MS-based methods, these are determined by reference to internal metabolite standards. The abundance of any molecular ion species typically carries information on concentration, but ion abundance is confounded by a number of features, including instrument response factors, ionization efficiency of the molecule, stability of the molecular ion species and the presence of other molecules that could cause ion suppression of the metabolite of interest. Thus, internal standards have been developed that can be used to generate appropriate calibration curves to convert abundance of ions into a quantitative measure of metabolite amount, level or concentration.

Non-limiting examples of internal standards include, but are not limited to, metabolite standards labelled with stable isotope-labelled versions of at least one of the metabolites to be quantified, which have a similar extraction recovery, ionization response and a similar chromatographic retention time; compound analogues of at least one of the metabolites to be quantified which are similar to the metabolites to be quantified but slightly different by parent mass; or chlorinated versions of at least one of the metabolites to be quantified, which commonly have a similar chromatographic retention time.

The internal standard is typically added at a known concentration into every sample, including the standards, at the beginning of the sample preparation, typically before the plasma preparation or solid phase extraction. It will be appreciated by the one skilled in the art that the amount of the internal standard needs to be higher than the limit of quantitation but low enough to avoid a suppression of the ionization of the analyte. Based on the known concentration of the internal standard present in the sample, the measured values for the metabolite of interest can be quantified by interpolating the response ratio between the metabolite and the internal standard to a standard curve.

These methods for determining metabolite concentrations by reference to internal metabolite standards are well-known in the art and have been described, *e.g*., in Ciccimaro & Blair, 2010. Bioanalysis. 2(2):311-41; or Postle & Hunt, 2009. J Chromatogr B Analyt Technol Biomed Life Sci. 877(26):2716-21.

Preferably, the internal metabolite standards are stable isotope-labelled standards. As used herein, the term **"stable",** when referring to isotopes, means that the isotopes are not radioactive, *i.e.,* they do not decay spontaneously. Metabolite standards labelled with one or more stable isotopes are stable isotope-labelled versions of the metabolite to be quantified and are well-known in the art. Typically, the isotopes employed are stable isotopes of carbon, nitrogen or hydrogen, such as, *e.g*., ¹²C and ¹³C, ¹⁴N and ¹⁵N and ²H. Such stable isotope-labelled metabolite standards have been described, *e.g.,* in Lee et al., 2010. Clin Biochem. 43(16-17): 1269-77.

In one embodiment, the metabolic signature of glaucoma of the present invention comprises at least one metabolite selected from the group of biogenic amines, amino acids, fatty acid esters, carbohydrates and glycerophospholipids whose amount, level or concentration is upregulated or downregulated in a patient population.

The term **"patient population",** as used herein, refers to a population comprising at least 1, preferably at least 5, at least 10, at least 15, at least 20, at least 25, at least 30, at least 35, at least 40, at least 45, at least 50, at least 75, at least 100 or more subjects diagnosed with glaucoma, preferably with POAG, *i.e.,* of known glaucoma outcome. By implying a multitude of samples from the patient population, it is conceivable to calculate a mean or median amount, level or concentration for each metabolite respectively, and optionally, the variance for patient population.

In one embodiment, the decision as to whether a certain metabolite is upregulated or downregulated in a patient population is taken in comparison to a "reference population".

The term **"reference population",** as used herein, refers to a population comprising at least 1, preferably at least 5, at least 10, at least 15, at least 20, at least 25, at least 30, at least 35, at least 40, at least 45, at least 50, at least 75, at least 100 or more substantially healthy subjects, *i.e.,* subject who are not affected and/or who have not been diagnosed with glaucoma, preferably with POAG.

In one embodiment, the patient population and the reference population comprise substantially the same number of subjects. In one embodiment, the number of subjects in the patient population is at most three times bigger, at most twice bigger, at most 1.9 times bigger, at most 1.8 times bigger, at most 1.7 times bigger, at most 1.6 times bigger, at most 1.5 times bigger, at most 1.4 times bigger, at most 1.3 times bigger, at most 1.2 times bigger, at most 1.1 times bigger than the number of subjects in the reference population. In one embodiment, the number of subjects in the patient population is at most three times smaller, at most twice smaller, at most 1.9 times smaller, at most 1.8 times smaller, at most 1.7 times smaller, at most 1.6 times smaller, at most 1.5 times smaller, at most 1.4 times smaller, at most 1.3 times smaller, at most 1.2 times smaller, at most 1.1 times smaller than the number of subjects in the reference population.

The metabolic signature of glaucoma of the present invention was identified by mathematical modelling, and other methods of statistical and structural classifications of the amount, level or concentration of metabolites in samples from subjects with known glaucoma diagnosis (*i.e*., from both the patient population and reference population). Methods for determining metabolites whose amount, level or concentration is upregulated or downregulated in a patient population as compared to a reference population are well-known to the skilled artisan (Worley & Powers, 2013. Curr Metabolomics. 1(1):92-107 ; Gromski et al., 2015. Anal Chim Acta. 879:10-23; Giacomoni et al., 2015. Bioinformatics. 31(9):1493-5 ; Thévenot et al., 2015. J Proteome Res. 14(8):3322-35) and include, without limitation, comparing the level of metabolites in samples from subjects with known glaucoma diagnosis.

Such methods include, but are not limited to, univariate analysis (using, *e.g*., bilateral Student's t-test, non-parametric Mann-Whitney-Wilcoxon test, Fisher's exact test and the like) and multivariate analysis (using, *e.g.*, LASSO models; logistic regression models; PLS-R - partial least squares regression; PC-DFA - principal component-discriminant function analysis; PLSDA - partial least squares-discriminant analysis (including orthogonal and multilevel PLSDA); and the like).

In one embodiment, the metabolic signature of glaucoma of the present invention is a mathematical representation (including without limitation, the values, a mean, a median, a logistic regression model or a LASSO model) of the amount, level or concentration of the metabolites of the metabolic signature.

In one embodiment, a metabolite is considered as having an upregulated or downregulated amount, level or concentration in a patient population as compared to a reference population if, in a univariate analysis, the p-value, preferably the p-value after False Discovery Rate (FDR) correction, is at or below 0.05, preferably at or below 0.04, 0.03, 0.02, 0.01, 0.009, 0.008, 0.007, 0.006, 0.005 or below.

In one embodiment, a metabolite is considered as having an upregulated or downregulated amount, level or concentration in a patient population as compared to a reference population if, in a multivariate analysis, the -Log₁₀(*p*-value) is at or above 50, preferably at or above 60, 70, 80, 90, 100, 125, 150, 175, 200 or more; and/or if the absolute value of the logistic regression coefficient is at or above 0.02, preferably at or above 0.03, 0.04, 0.05, 0.06, 0.07, 0.08, 0.09, 0.1 or more.

In one embodiment, the metabolic signature of glaucoma of the present invention is indicative of glaucoma. In one embodiment, the metabolic signature of glaucoma of the present invention is indicative of being at risk of developing glaucoma.

In one embodiment, the metabolic signature of glaucoma of the present invention is indicative of glaucoma or of being at risk of developing glaucoma if the amount, level or concentration of at least at 1 metabolite, preferably 2 metabolites selected from the group comprising or consisting of octadecadienoylcarnitine and spermidine in a patient population is lower than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2 metabolites selected from the group comprising or consisting of decenoylcarnitine and PC ae 40:1 in a patient population is higher than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2 metabolites selected from the group comprising or consisting of octadecadienoylcarnitine and spermidine in a patient population is lower and if the amount, level or concentration of at least 1 metabolite, preferably 2 metabolites selected from the group comprising or consisting of decenoylcarnitine and PC ae 40:1 in a patient population is higher than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2 or 3 metabolites selected from the group comprising or consisting of octadecadienoylcarnitine, spermidine and spermine in a patient population is lower than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2, 3 or 4 metabolites selected from the group comprising or consisting of hexoses, methionine, PC ae 40:1 and tyrosine in a patient population is higher than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2 or 3 metabolites selected from the group comprising or consisting of octadecadienoylcarnitine, spermidine and spermine in a patient population is lower and if the amount, level or concentration of at least 1 metabolite, preferably 2, 3 or 4 metabolites selected from the group comprising or consisting of hexoses, methionine, PC ae 40:1 and tyrosine in a patient population is higher than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2, 3 or 4 metabolites selected from the group comprising or consisting of histamine, octadecadienoylcarnitine, spermidine and spermine in a patient population is lower than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2, 3, 4, 5, 6, 7 or 8 metabolites selected from the group comprising or consisting of arginine, butyrylcarnitine, decenoylcarnitine, dodecenoylcarnitine, hexoses, methionine, PC ae 40:1 and tyrosine in a patient population is higher than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2, 3 or 4 metabolites selected from the group comprising or consisting of histamine, octadecadienoylcarnitine, spermidine and spermine in a patient population is lower and if the amount, level or concentration of at least 1 metabolite, preferably 2, 3, 4, 5, 6, 7 or 8 metabolites selected from the group comprising or consisting of arginine, butyrylcarnitine, decenoylcarnitine, dodecenoylcarnitine, hexoses, methionine, PC ae 40:1 and tyrosine in a patient population is higher than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2, 3 or 4 metabolites selected from the group comprising or consisting of octadecadienoylcarnitine, octadecenoylcarnitine, spermidine and spermine in a patient population is lower than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2, 3, 4, 5, 6, 7, 8 or 9 metabolites selected from the group comprising or consisting of hexoses, methionine, methionine sulfoxide, PC aa 34:2, PC aa 34:4, PC aa 36:4, PC ae 40:1, propionylcarnitine and tyrosine in a patient population is higher than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2, 3 or 4 metabolites selected from the group comprising or consisting of octadecadienoylcarnitine, octadecenoylcarnitine, spermidine and spermine in a patient population is lower and if the amount, level or concentration of at least 1 metabolite, preferably 2, 3, 4, 5, 6, 7, 8 or 9 metabolites selected from the group comprising or consisting of hexoses, methionine, methionine sulfoxide, PC aa 34:2, PC aa 34:4, PC aa 36:4, PC ae 40:1, propionylcarnitine and tyrosine in a patient population is higher than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2, 3, 4 or 5 metabolites selected from the group comprising or consisting of histamine, octadecadienoylcarnitine, octadecenoylcarnitine, spermidine and spermine in a patient population is lower than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2, 3, 4, 5, 6, 7, 8, 9, 10, 11, 12 or 13 metabolites selected from the group comprising or consisting of arginine, butyrylcarnitine, decenoylcarnitine, dodecenoylcarnitine, hexoses, methionine, methionine sulfoxide, PC aa 34:2, PC aa 34:4, PC aa 36:4, PC ae 40:1, propionylcarnitine and tyrosine in a patient population is higher than the amount, level or concentration of the same metabolite in a reference population.

In one embodiment, the metabolic signature of glaucoma of the present invention is indicative of glaucoma or of being at risk of developing glaucoma if the amount, level or concentration of at least 1 metabolite, preferably 2, 3, 4 or 5 metabolites selected from the group comprising or consisting of histamine, octadecadienoylcarnitine, octadecenoylcarnitine, spermidine and spermine in a patient population is lower and if the amount, level or concentration of at least 1 metabolite, preferably 2, 3, 4, 5, 6, 7, 8, 9, 10, 11, 12 or 13 metabolites selected from the group comprising or consisting of arginine, butyrylcarnitine, decenoylcarnitine, dodecenoylcarnitine, hexoses, methionine, methionine sulfoxide, PC aa 34:2, PC aa 34:4, PC aa 36:4, PC ae 40:1, propionylcarnitine and tyrosine in a patient population is higher than the amount, level or concentration of the same metabolite in a reference population.

Another object of the present invention is a method for diagnosing glaucoma in a subject. Another object of the present invention is a method for identifying a subject as being at risk of developing glaucoma. Another object of the present invention is a method for identifying a predisposition for developing glaucoma in a subject. Another object of the present invention is a method for stratifying a subject affected with glaucoma. Another object of the present invention is a method for stratifying a subject at risk of being affected with glaucoma.

In one embodiment of the methods according to the present invention, glaucoma is open-angle glaucoma (OAG). In a preferred embodiment of the methods according to the present invention, glaucoma is POAG.

In one embodiment, the subject is an animal, preferably a mammal, more preferably a primate. In one embodiment, the subject is a human.

In one embodiment, the subject is a male. In one embodiment, the subject is female.

In one embodiment, the subject is a child, an adolescent or an adult.

In one embodiment, the subject is above the age of 40 years. In one embodiment, the subject is above the age of 50 years. In one embodiment, the subject is above the age of 60 years. In one embodiment, the subject is above the age of 70 years. In one embodiment, the subject is above the age of 80 years or more.

In one embodiment, the subject is aged from 0 to 20 years old. In one embodiment, the subject is aged from 20 to 40 years old. In one embodiment, the subject is aged from 40 to 50 years old. In one embodiment, the subject is aged from 50 to 55 years old. In one embodiment, the subject is aged from 55 to 60 years old. In one embodiment, the subject is aged from 60 to 65 years old. In one embodiment, the subject is aged from 65 to 70 years old. In one embodiment, the subject is aged from 70 to 75 years old. In one embodiment, the subject is aged from 75 to 80 years old. In one embodiment, the subject is aged from 80 to 85 years old or more.

In one embodiment, the subject is/was not diagnosed with glaucoma. In one embodiment, the subject is at risk of being diagnosed with glaucoma. In one embodiment, the subject is/was diagnosed with glaucoma.

Current methods for diagnosing glaucoma or POAG include, but are not limited to, tonometry, ophthalmoscopy, optical coherence tomography, gonioscopy, pachymetry, perimetry and the like.

In one embodiment, a subject is considered as being at risk of being diagnosed with glaucoma, if said subject meets at least one, at least two, at least three or more of the following criteria: eye injury, familial history of glaucoma, genetic predisposition to glaucoma, ethnicity, environmental risks (including, *e.g*., diet or smoking) and clinical factors (including, *e.g*., elevated IOP, hypertension, myopia, hyperopia, thin cornea and diabetes).

In one embodiment, the subject was not previously treated for glaucoma. In one embodiment, the subject was previously treated for glaucoma. In one embodiment, the subject is currently treated for glaucoma.

Examples of glaucoma treatments include, but are not limited to, eye drops (including, *e.g.,* IOP lowering eye drops), laser surgery, trabeculectomy, trabeculoplasty, ultrasonography, photodynamic therapy, placement of glaucoma tube shunts, no-stitch cataract surgery and combinations thereof.

IOP lowering eye drops are often the first choice for treating subjects. Such medications decrease eye pressure by helping the eye's fluid to drain better and/or by decreasing the amount of fluid made by the eye. IOP lowering eye drops may comprise any or several of the following: prostaglandin analogues (including, but not limited to, latanoprost, bimatoprost, travoprost and tafluprost), beta blockers (including, but not limited to, timolol), alpha agonists (including, but not limited to, brimonidine and iopidine), carbonic anhydrase inhibitors (including, but not limited to, dorzolamide, brinzolamide, acetazolamide and methazolamide) and cholinergic agonists.

In one embodiment, the methods according to the present invention comprise a step of providing a sample from a subject.

In one embodiment, the sample is a bodily fluid. Examples of bodily fluids include, but are not limited to, blood, plasma, serum, lymph, ascetic fluid, cystic fluid, urine, bile, nipple exudate, synovial fluid, bronchoalveolar lavage fluid, sputum, amniotic fluid, peritoneal fluid, cerebrospinal fluid, pleural fluid, pericardial fluid, semen, saliva, sweat and alveolar macrophages.

In one embodiment, the sample is a serum sample. In a preferred embodiment, the sample is a blood sample. In a more preferred embodiment, the sample is a plasma sample.

In one embodiment, the sample was previously taken from the subject, *i.e.,* the methods of the invention do not comprise a step of recovering a sample from the subject. Consequently, according to this embodiment, the methods of the invention are non-invasive methods, *i.e.,* the methods of the invention are *in vitro* methods.

In one embodiment, the sample was previously taken from the subject after a fasting period.

In one embodiment, the fasting period is of about 12 hours (such as, *e.g*., overnight fasting, *i.e.,* before breakfast), of about 6 hours, preferably of about 3 hours before sampling.

In one embodiment, the sample was previously taken from the subject into a suitable container, such as, *e.g.,* in a S-Monovette® serum tube, preferably in a S-Monovette® 2.7 mL potassium-EDTA serum tube.

In one embodiment, the methods according to the present invention comprise a step of preparing the sample.

In one embodiment, the sample container is gently but thoroughly shaked.

In one embodiment where the sample is blood, cells and plasma are separated, *e.g.,* by centrifugation (such as, *e.g.,* by centrifugation at 20-24°C, for 10 minutes at 2500 g).

In one embodiment, the methods according to the present invention further comprise a step of determining a personalized metabolic signature in a sample from the subject.

In one embodiment, determining a personalized metabolic signature in a sample from the subject comprises assessing or measuring the amount, level or concentration of at least one metabolite selected from the group of biogenic amines, at least one metabolite selected from the group of amino acids, at least one metabolite selected from the group of fatty acid esters, at least one metabolite selected from the group of carbohydrates and/or at least one metabolite selected from the group of glycerophospholipids.

The amount, level or concentration of a metabolite is assessed or measured by physical methods, chemical methods or a combination thereof, which are well-known in the art. Such methods include, but are not limited to, mass spectrometry, liquid chromatography, Raman spectroscopy, emission spectroscopy, absorbance spectroscopy, circular dichroism spectroscopy, nuclear magnetic resonance (NMR), thin-layer chromatography, affinity chromatography, gas chromatography, size-exclusion chromatography, and combinations thereof.

In one embodiment, the amount, level or concentration of a metabolite is measured by flow injection analysis coupled with tandem mass spectrometry (FIA-MS/MS). In one embodiment, the amount, level or concentration of a metabolite is measured by liquid chromatography coupled with mass spectrometry (LC-MS).

In one embodiment, determining a personalized metabolic signature in a sample from the subject comprises measuring the amount, level or concentration of at least 1 metabolite, preferably 2, 3 or 4 metabolites selected from the group comprising or consisting of decenoylcarnitine, octadecadienoylcarnitine, PC ae 40:1 and spermidine.

In one embodiment, determining a personalized metabolic signature in a sample from the subject comprises measuring the amount, level or concentration of at least 1 metabolite, preferably 2, 3, 4, 5, 6 or 7 metabolites selected from the group comprising or consisting of hexoses, methionine, octadecadienoylcarnitine, PC ae 40:1, spermidine, spermine and tyrosine.

In one embodiment, determining a personalized metabolic signature in a sample from the subject comprises measuring the amount, level or concentration of at least 1 metabolite, preferably 2, 3, 4, 5, 6, 7, 8, 9, 10, 11 or 12 metabolites selected from the group comprising or consisting of arginine, butyrylcarnitine, decenoylcarnitine, dodecenoylcarnitine, hexoses, histamine, methionine, octadecadienoylcarnitine, PC ae 40:1, spermidine, spermine and tyrosine.

In one embodiment, determining a personalized metabolic signature in a sample from the subject comprises measuring the amount, level or concentration of at least 1 metabolite, preferably 2, 3, 4, 5, 6, 7, 8, 9, 10, 11, 12 or 13 metabolites selected from the group comprising or consisting of hexoses, methionine, methionine sulfoxide, octadecadienoylcarnitine, octadecenoylcarnitine, PC aa 34:2, PC aa 34:4, PC aa 36:4, PC ae 40:1, propionylcarnitine, spermidine, spermine and tyrosine.

In one embodiment, determining a personalized metabolic signature in a sample from the subject comprises measuring the amount, level or concentration of at least 1 metabolite, preferably 2, 3, 4, 5, 6, 7, 8, 9, 10, 11, 12, 13, 14, 15, 16, 17 or 18 metabolites selected from the group comprising or consisting of arginine, butyrylcarnitine, decenoylcarnitine, dodecenoylcarnitine, hexoses, histamine, methionine, methionine sulfoxide, octadecadienoylcarnitine, octadecenoylcarnitine, PC aa 34:2, PC aa 34:4, PC aa 36:4, PC ae 40:1, propionylcarnitine, spermidine, spermine and tyrosine.

In one embodiment, the methods according to the present invention comprise a step of preparing a hard and/or soft copy comprising the values of the amount, level or concentration of measured metabolites.

Examples of hard copies include, but are not limited to, print-outs, hand-written information, photographs, data as originally obtained (such as, *e.g.,* from a mass spectrometer).

Examples of soft copies include, but are not limited to, any form of computer readable files such as, *e.g.,* the originally obtained data output from the machine performing the measurements (*e.g*., a mass spectrometer) or from the respective analysis program; word or other text software documents containing the values; screen shots.

It will be appreciated that the values comprised in said hard or soft copy can, *e.g.,* be raw values (*i.e.,* original data as obtained) or calculated values (*e.g*., in the form of numerical values derived from the measurements).

In one embodiment, the methods of the present invention comprise a step of comparing the personalized metabolic signature with the metabolic signature of glaucoma according to the present invention.

In one embodiment, said comparison may include a comparison of the amount, level or concentration of at least 1 metabolite, preferably at least 2, 3, 4, 5, 6, 7, 8, 9, 10, 11, 12, 13, 14, 15, 16, 17 or 18 metabolites of the personalized metabolic signature with the amount, level or concentration of the same at least 1 metabolite, preferably at least 2, 3, 4, 5, 6, 7, 8, 9, 10, 11, 12, 13, 14, 15, 16, 17 or 18 metabolites of the metabolic signature of glaucoma according to the present invention.

In one embodiment, said comparison may include a comparison of whether the amount, level or concentration of at least 1 metabolite, preferably at least 2, 3, 4, 5, 6, 7, 8, 9, 10, 11, 12, 13, 14, 15, 16, 17 or 18 metabolites of the personalized metabolic signature is qualitatively and/or quantitatively the same as the amount, level or concentration of the same at least 1 metabolite, preferably at least 2, 3, 4, 5, 6, 7, 8, 9, 10, 11, 12, 13, 14, 15, 16, 17 or 18 metabolites of the metabolic signature of glaucoma according to the present invention.

In one embodiment, said comparison may include a comparison of whether the amount, level or concentration of at least 1 metabolite, preferably at least 2, 3, 4, 5, 6, 7, 8, 9, 10, 11, 12, 13, 14, 15, 16, 17 or 18 metabolites of the personalized metabolic signature is greater than, equal to, or less than a threshold value.

In one embodiment, said comparison of the amount, level or concentration of at least 1 metabolite, preferably at least 2, 3, 4, 5, 6, 7, 8, 9, 10, 11, 12, 13, 14, 15, 16, 17 or 18 metabolites of the personalized metabolic signature may include a comparison "by eye".

In one embodiment, said comparison of the amount, level or concentration of at least 1 metabolite, preferably at least 2, 3, 4, 5, 6, 7, 8, 9, 10, 11, 12, 13, 14, 15, 16, 17 or 18 metabolites of the personalized metabolic signature may include one or more forms of statistical, mathematical or physiological analysis of the amount, level or concentration of the same at least 1 metabolite, preferably at least 2, 3, 4, 5, 6, 7, 8, 9, 10, 11, 12, 13, 14, 15, 16, 17 or 18 metabolites of the metabolic signature of glaucoma according to the present invention.

Examples of such statistical, mathematical or physiological analysis include, but are not limited to, regression analysis, univariate analysis, multivariate analysis, variation calculations, best-fit analysis, curve fitting, extrapolation, interpolation, least squares, mean calculations, simulation analysis.

In one embodiment, said comparison comprises or consists of comparing a statistical or mathematical representation (such as, *e.g*., values, mean, median, or regression) of the amount, level or concentration of at least 1 metabolite, preferably at least 2, 3, 4, 5, 6, 7, 8, 9, 10, 11, 12, 13, 14, 15, 16, 17 or 18 metabolites of the personalized metabolic signature with a statistical or mathematical representation (such as, *e.g.,* values, mean, median, or regression) of the amount, level or concentration of at least 1 metabolite, preferably at least 2, 3, 4, 5, 6, 7, 8, 9, 10, 11, 12, 13, 14, 15, 16, 17 or 18 metabolites of the metabolic signature of glaucoma according to the present invention.

In one embodiment, said comparison comprises or consists of determining a correlation between a statistical or mathematical representation (such as, *e.g.,* values, mean, median, or regression) of the amount, level or concentration of at least 1 metabolite, preferably at least 2, 3, 4, 5, 6, 7, 8, 9, 10, 11, 12, 13, 14, 15, 16, 17 or 18 metabolites of the personalized metabolic signature and a statistical or mathematical representation (such as, e.g., values, mean, median, or regression) of the amount, level or concentration of at least 1 metabolite, preferably at least 2, 3, 4, 5, 6, 7, 8, 9, 10, 11, 12, 13, 14, 15, 16, 17 or 18 metabolites of the metabolic signature of glaucoma according to the present invention.

In one embodiment, said comparison comprises or consists of determining a difference between a statistical or mathematical representation (such as, e.g., values, mean, median, or regression) of the amount, level or concentration of at least 1 metabolite, preferably at least 2, 3, 4, 5, 6, 7, 8, 9, 10, 11, 12, 13, 14, 15, 16, 17 or 18 metabolites of the personalized metabolic signature and a statistical or mathematical representation (such as, *e.g.,* values, mean, median, or regression) of the amount, level or concentration of at least 1 metabolite, preferably at least 2, 3, 4, 5, 6, 7, 8, 9, 10, 11, 12, 13, 14, 15, 16, 17 or 18 metabolites of the metabolic signature of glaucoma according to the present invention.

In one embodiment, the difference between the personalized metabolic signature and the metabolic signature of glaucoma according to the present invention is defined as statistically significant with p-value at or below 0.05, preferably at or below 0.01, more preferably at or below 0.005.

In one embodiment, the difference between the personalized metabolic signature and the metabolic signature of glaucoma according to the present invention is defined a statistically significant difference in concentration, level or amount of at least 1, preferably at least 2, 3, 4, 5, 6 ,7, 8, 9, 10, 11, 12, 13, 14, 15, 16, 17 or 18 metabolites with p-value at or below 0.05, preferably at or below 0.01, more preferably at or below 0.006.

In one embodiment, appreciation of the difference between the personalized metabolic signature and the metabolic signature of glaucoma according to the present invention is left to the physician who is able to interpret a personalized metabolic signature, in particular the concentration, level or amount of at least 1, preferably at least 2, 3, 4, 5, 6,7, 8, 9, 10, 11, 12, 13, 14, 15, 16, 17 or 18 metabolites, with the metabolic signature according to the present invention.

In one embodiment, the methods of the present invention comprise a step of diagnosing the subject as being affected or as being at risk of being affected with glaucoma.

In one embodiment, the subject is diagnosed as being affected or as being at risk of being affected with glaucoma based at least in part upon a substantial similarity between the metabolic signature in a sample from the subject and the metabolic signature according to the present invention.

In one embodiment, the subject is diagnosed as being affected or as being at risk of being affected with glaucoma when the metabolic signature in a sample from the subject is substantially similar to the metabolic signature according to the present invention.

The terms "substantially similar" and "substantial similarity", as used herein, denotes a sufficiently high degree of similarity between two signatures

In one embodiment, the methods of the invention comprise the steps of
a) providing a sample from the subject,
b) determining a personalized metabolic signature in said sample from the subject,
c) comparing the personalized metabolic signature provided in step b) with the metabolic signature of glaucoma according to the present invention, and
d) diagnosing the subject as being affected or as being at risk of being affected with glaucoma when the comparison of step c) reveals that the personalized metabolic signature provided in step b) is comparable to the metabolic signature of glaucoma according to the present invention.

The present invention also relates to a method for treating a subject in need thereof, comprising the steps of
a) providing a sample from the subject,
b) determining a personalized metabolic signature in said sample from the subject,
c) comparing the personalized metabolic signature provided in step b) with the metabolic signature of glaucoma according to the present invention,
d) diagnosing the subject as being affected with glaucoma when the comparison of step c) reveals that the personalized metabolic signature provided in step b) is comparable to the metabolic signature of glaucoma according to the present invention, and
e) treating the subject for glaucoma.

Examples of glaucoma treatments include, but are not limited to, eye drops (including, *e.g.,* IOP lowering eye drops), laser surgery, trabeculectomy, trabeculoplasty, ultrasonography, photodynamic therapy, placement of glaucoma tube shunts, no-stitch cataract surgery and combinations thereof.

IOP lowering eye drops are often the first choice for treating subjects. Such medications decrease eye pressure by helping the eye's fluid to drain better and/or by decreasing the amount of fluid made by the eye. IOP lowering eye drops may comprise any or several of the following: prostaglandin analogues (including, but not limited to, latanoprost, bimatoprost, travoprost and tafluprost), beta blockers (including, but not limited to, timolol), alpha agonists (including, but not limited to, brimonidine and iopidine), carbonic anhydrase inhibitors (including, but not limited to, dorzolamide, brinzolamide, acetazolamide and methazolamide) and cholinergic agonists.

The present invention also relates to a kit for measuring the amount, level or concentration of at least one metabolite of the metabolic signature according to the present invention and/or for implementing the methods of the present invention.

In one embodiment, the kit comprises means for determining the amount, level or concentration of at least one metabolite, preferably of at least 2, 3, 4, 5, 6, 7, 8, 9, 10, 11, 12, 13, 14, 15, 16, 17 or 18 metabolites of the metabolic signature according to the present invention.

In one embodiment, the kit further comprises at least one stable isotope-labelled metabolite, preferably of at least 2, 3, 4, 5, 6, 7, 8, 9, 10, 11, 12, 13, 14, 15, 16, 17 or 18 stable isotope-labelled metabolites.

In one embodiment, the kit comprises stable isotope-labelled arginine, stable isotope-labelled butyrylcarnitine, stable isotope-labelled decenoylcarnitine, stable isotope-labelled dodecenoylcarnitine, stable isotope-labelled hexoses, stable isotope-labelled histamine, stable isotope-labelled methionine, stable isotope-labelled methionine sulfoxide, stable isotope-labelled octadecadienoylcarnitine, stable isotope-labelled octadecenoylcarnitine, stable isotope-labelled PC aa 34:2, stable isotope-labelled PC aa 34:4, stable isotope-labelled PC aa 36:4, stable isotope-labelled PC ae 40:1, stable isotope-labelled propionylcarnitine, stable isotope-labelled spermidine, stable isotope-labelled spermine, stable isotope-labelled tyrosine, and/or stable isotope-labelled isobaric metabolites having the same molecular mass as arginine, butyrylcarnitine, decenoylcarnitine, dodecenoylcarnitine, hexoses, histamine, methionine, methionine sulfoxide, octadecadienoylcarnitine, octadecenoylcarnitine, PC aa 34:2, PC aa 34:4, PC aa 36:4, PC ae 40:1, propionylcarnitine, spermidine, spermine and/or tyrosine, but different chemical formula.

In one embodiment where the amount, level or concentration of metabolites is determined by mass spectrometry (MS), the kit further comprises means for preparing the sample for MS analysis.

In one embodiment where the amount, level or concentration of metabolites is determined by chromatography, the kit further comprises means for preparing the sample for chromatography analysis.

In one embodiment where the amount, level or concentration of metabolites is determined by chromatography coupled with MS, the kit further comprises means for preparing the sample for chromatography coupled with MS analysis.

In one embodiment where the amount, level or concentration of metabolites is determined by NMR, the kit further comprises means for preparing the sample for NMR analysis.

In one embodiment where the amount, level or concentration of metabolites is determined using a combination of the above-mentioned techniques, the kit further comprises means for preparing the sample for said combinatorial analysis.

In one embodiment, the means for preparing the sample comprise internal standards and/or control for measuring (and validating the measurement) of the amount, level or concentration of metabolites.

In one embodiment, the kit further comprises an analysis software for determining the amount, level or concentration of metabolites. In one embodiment, the kit comprises an analysis software for performing the methods of the present invention. In one embodiment, said software comprises the metabolic signature according to the present invention. In one embodiment, said software provides a score based on the methods of the invention used alone, or in combination with other methods.

In one embodiment, the kit comprises a user manual for the use thereof.

### BRIEF DESCRIPTION OF THE DRAWINGS

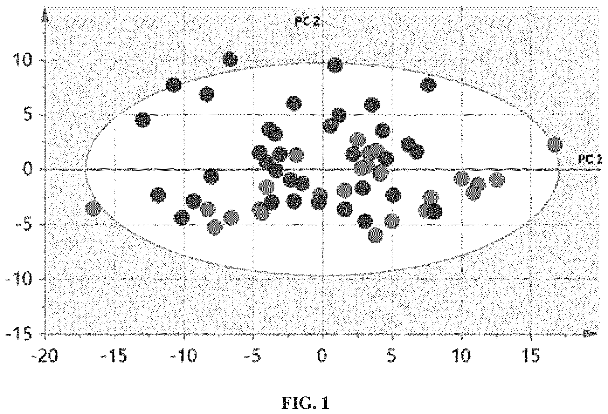

**Figure 1** is a PCA scatter plot obtained from the matrix of metabolites for the 35 patients suffering from glaucoma (dark grey dots) and the 27 controls (light grey dots). There is no clear grouping, nor are there any outliers (according to Hotelling's T2 range). PC 1, PC 2: principal components 1 and 2.

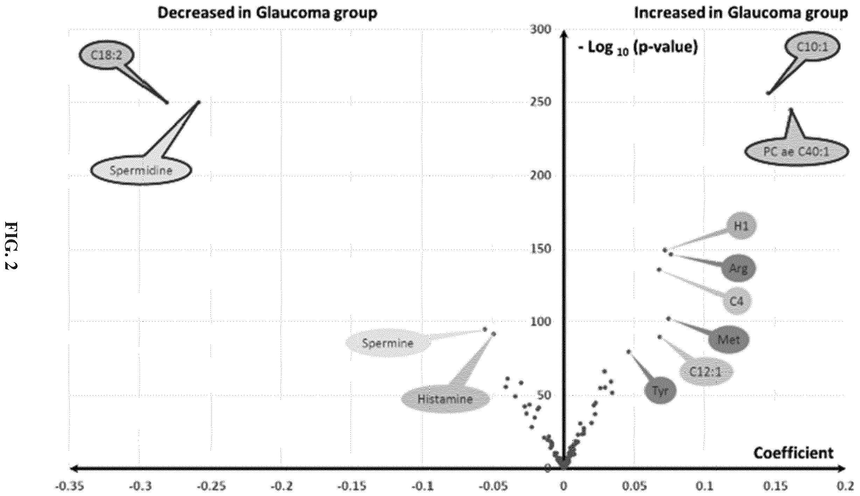

**Figure 2** is Volcano plot showing the estimated mean coefficients for the thousand LASSO models and the -log₁₀ (*p*-values) obtained by testing the nullity of the mean of each of the 151 coefficients using this iterative process. Only the most important metabolites are labelled. Four metabolites, highlighted with a red border, are clearly distinguished from the other metabolites. Arg: arginine; Met: methionine; Tyr: tyrosine; H1: hexose; C4: butyrylcarnitine; C10:1: decenoylcarnitine; C12:1: dodecenoylcarnitine; C18:2: octadecadienoylcarnitine; and PC ae 40:1: acyl-alkyl phosphatidylcholine 40:1.

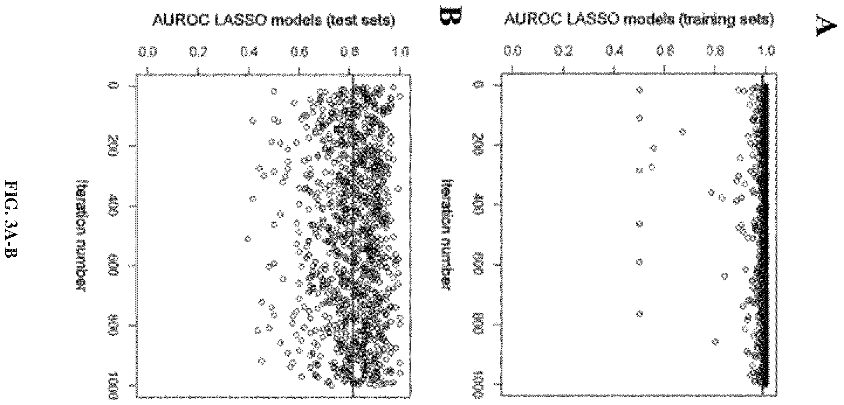

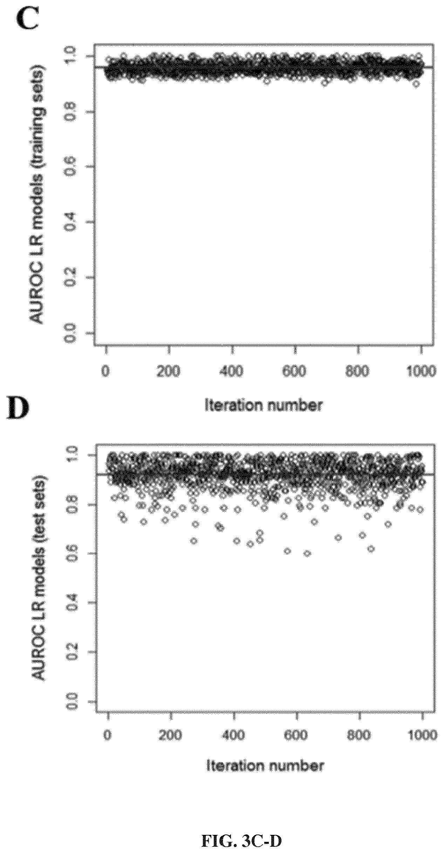

**Figure 3** shows the area calculated under the receiving operator characteristic curve (AUROC) for thousand LASSO (**A** and **B**) and logistic regression (LR) (**C** and **D**) models.

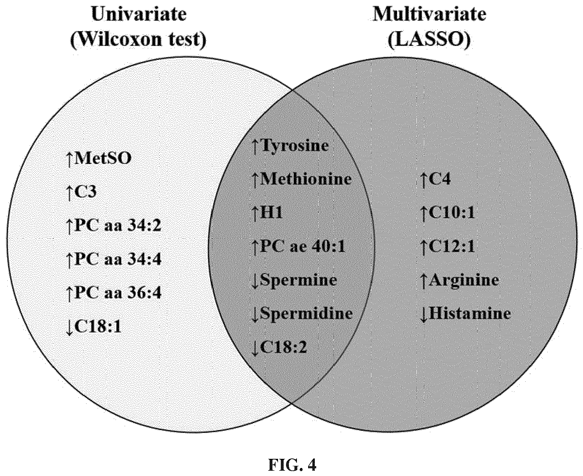

**Figure 4** is a Venn diagram showing the most important metabolites found after univariate analysis (light grey ellipse) and multivariate analysis (dark grey ellipse). At the intersection of the two ellipses are the metabolites which were commonly found after univariate analysis and multivariate analysis. H1: hexoses; C3: propionylcarnitine; C4: butyrylcarnitine; C10:1: decenoylcarnitine; C12:1: dodecenoylcarnitine; C18:1: octadecenoylcarnitine; C18:2: octadecadienoylcarnitine; MetSO: methionine sulfoxide; PC aa 32:2, 34:4, 36:4: phosphatidylcholine diacyl 32:2, 34:4 and 36:4, respectively; and PC ae 40:1: phosphatidylcholine acyl-alkyl 40:1. The arrows indicate the comparative amount, level or concentration of each metabolite with respect to the amount, level or concentration of the same metabolite in a population of substantially healthy subjects (↑: increased amount, level or concentration; ↓: decreased amount, level or concentration).

### EXAMPLES

The present invention is further illustrated by the following examples.

### Example 1: Clinical features of POAG patients and controls

### Materials and Methods

### Ethics Statement

Participants were included after having given their informed written consent for the research. The study, conducted in accordance with the ethical standards set forth in the Helsinki Declaration (1983), was approved by the University of Angers Ethical Review Committee (CPP OUEST 2, agreement number: CB 2013-04).

### Study participants

Individuals were recruited from the Department of Ophthalmology of Angers University Hospital, France.

The diagnosis of POAG was based on consensual criteria, *i.e.,* intraocular pressure > 21 mmHg, and glaucomatous optic nerve damage with progressive optic disc cupping (Weinreb et al., 2004. Lancet. 363(9422):1711-20). All patients had open iridocorneal angles as determined by gonioscopic examination. Standard automated perimetry (Humphrey field analyzer, Carl Zeiss, Dublin, CA, USA) with the 24-2 SITA Fast algorithm was performed on all the POAG patients, and values of the visual field mean defect (VF-MD) were used to grade the severity of POAG as "mild" with values lower than -6dB, "moderate" with values between -6dB and -12dB, and "severe" with values higher than -12dB (perimetric Hoddap-Parrish-Anderson criteria). The reliability indices retained were false positive or false negative rates under 15%, and fixation losses under 20%.

Other additional tests performed on patients with POAG included evaluation of the retinal nerve fiber layer (RNFL) thickness using spectral domain optical coherence tomography (OCT) and measurement of the central corneal thickness (CCT), (Cirrus OCT, Carl Zeiss Meditec, Dublin, CA, USA). The best-corrected visual acuity was measured using the decimal Monoyer charts, with the results converted into logMAR units for statistical analysis. Intraocular pressures were measured using the Goldmann applanation tonometer. The history of the glaucoma treatment was documented.

Control subjects were sex- and age-matched individuals selected among patients receiving cataract surgery at the same Department of Ophthalmology. The inclusion criteria were: visual acuity ≥ 20/50 and the absence of any other associated ocular conditions excepting cataract. The exclusion criteria were based on personal reasons or because of a family history of glaucoma, ocular hypertension or any other intraocular pathology, including retinal disorders. The control group (n = 27) was age- and sex-matched with the POAG group of patients (n = 36).

### Sample collection

Fasting blood samples were collected in heparin tubes in the Department of Ophthalmology, rapidly transported in crushed ice to the Hospital Biological Resources Center, and immediately centrifuged for 10 minutes at 3000 g at +4°C before recovery of the supernatant (plasma), which was conserved at -80°C in 500 µL aliquots until metabolomics analysis.

### Statistical analysis

Univariate analysis of clinical data was carried out using the bilateral Student's t-test, with differences being considered significant at p-values ≤ 0.05.

### Results

Comparisons between individuals with POAG (n=36) and controls (n=27) in terms of demographic and environmental data, as well as comorbid medical conditions, are presented in **Table 1.** The mean age of individuals with POAG did not differ significantly from that of controls, nor did the sex ratio. There were no between-group differences regarding hypertension, hyperlipidemia, diabetes, or thyroid disease, but the body mass index (BMI) was significantly lower in individuals with POAG compared to the age- and sex-matched control group (p<0.01) **(Table 1).**

**Table 1. Demographic data and comorbidity status of individuals with POAG compared to controls. BMI: Body mass index (height/weight2).**

| | **POAG (n = 36)** | **Controls (n = 27)** | ***p*-values** |
|---|---|---|---|
| **Average age (y)** | 72.00 | 73.04 | 0.51 |
| **Females (%)** | 58.33 | 44.44 | 0.28 |
| **Mean BMI (kg/m²)** | 24.84 | 27.49 | ***<0.01*** |
| **Diabetes (%)** | 5.88 | 3.70 | 0.70 |
| **Hypertension (%)** | 44.44 | 62.96 | 0.15 |
| **Hyperlipidemia (%)** | 19.44 | 25.93 | 0.55 |
| **Thyroid disease (%)** | 19.44 | 11.11 | 0.36 |

There was no significant difference between the groups in term of systemic medications **(Table 2).**

**Table 2. Systemic medication of individuals with POAG compared to controls.**

| | **POAG (n = 36)** | **Controls (n = 27)** | ***p*-values** |
|---|---|---|---|
| **Antihypertensives (%)** | 44.44 | 66.66 | 0.08 |
| **Lipid-lowering medications (%)** | 19.44 | 25.93 | 0.55 |
| **Antiplatelet therapy/oral anticoagulation drugs (%)** | 13.88 | 29.63 | 0.15 |
| **Oral diabetes medications (%)** | 11.11 | 7.41 | 0.62 |
| **Insulin (%)** | 2.78 | 3.70 | 0.84 |
| **Corticosteroids (%)** | 0 | 7.41 | 0.16 |
| **Thyroid hormone (%)** | 16.67 | 11.11 | 0.53 |
| **Estrogen (%)** | 2.78 | 3.70 | 0.84 |
| **Vitamin D (%)** | 5.55 | 18.52 | 0.14 |
| **Others (%)** | 38.90 | 55.55 | 0.19 |

The general ophthalmological features of patients with POAG were also compared with those of controls (Table 3). Unsurprisingly, the POAG group had significantly thinner RNFL than controls as measured by OCT (p<0.001). In contrast, there was no significant difference between the POAG group and the controls regarding the intraocular pressure, which may have been affected by the treatment for glaucoma in the POAG group (p=0.60) and the central corneal thickness (p=0.09). Since the control group had received cataract surgery, the visual acuity was significantly better than in the POAG group (p=0.01). In the POAG group, 50% had mild POAG, 14% had moderate POAG, and 36% had severe POAG.

**Table 3. Ophthalmological features and glaucoma medication of individuals with POAG compared to controls. IOP: intraocular pressure; CCT: central corneal thickness; RNFL: retinal nerve fiber layer; VF-MD: visual field mean defect.**

| | | **POAG (n = 36)** | **Controls (n = 27)** | ***p-*values** |
|---|---|---|---|---|
| **Mean visual acuity (LogMar**) | | +0.12 | +0.04 | ***0.01*** |
| **IOP** (**mmHg**) | | 13.61 | 13.22 | 0.60 |
| **Mean CCT (µm)** | | 527 | 552 | 0.09 |
| **Average RNFL thickness (µm)** | | 65.27 | 87.00 | ***<0.001*** |
| **Mean VF-MD (dB)**, **(worse eye)** | | -7.84 | --- | --- |
| **Glaucoma severity (%)** | **Mild** | **50** | **---** | **---** |
| | **Moderate** | **14** | **---** | **---** |
| | **Severe** | 36 | --- | --- |
| **Glaucoma medications (%)** | **Beta-blockers** | 58 | --- | --- |
| | **Prostaglandin analogue** | 64 | --- | --- |
| | **Alpha-2-agonists** | 8 | --- | --- |
| | **Carbonic anhydrase inhibitor** | 30 | --- | --- |

### Example 2: Metabolomics analysis

### Material and Methods

### Metabolomics analysis

Targeted quantitative metabolomics analyses were carried out using the Biocrates® Absolute IDQ p180 kit (Biocrates Life sciences AG, Innsbruck, Austria). Used with mass spectrometry (QTRAP 5500, SCIEX, Villebon-sur-Yvette, France), this kit allows the quantification of up to 188 different endogenous molecules distributed as follows: free carnitine (C0), 39 acylcarnitines (C), the sum of hexoses (H1), 21 amino acids, 21 biogenic amines and 105 lipids. The lipids are distributed in the kit in four different classes: 14 lysophosphatidylcholines (lysoPC), 38 diacyl-phosphatidylcholines (PC aa), 38 acyl-alkyl-phosphatidylcholines (PC ae) and 15 sphingomyelins (SM). The full list of individual metabolites is available at http://www.biocrates.com/products/research-products/absoluteidq-p180-kit. Flow injection analysis coupled with tandem mass spectrometry (FIA-MS/MS) was used for the analysis of carnitine, acylcarnitines, lipids and hexoses. Liquid chromatography (LC) was used for separating amino acids and biogenic amines before quantitation with mass spectrometry.

All reagents used in this analysis were of LC-MS grade and purchased from VWR (Fontenay-sous-Bois, France) and Merck (Molsheim, France). Sample preparation and analysis were performed following the Biocrates Kit User Manual. Briefly, each plasma sample was thoroughly vortexed after thawing and centrifuged at 4°C for 5 minutes at 5000 g. Ten microliters of each sample were then added to the filter on the upper wells of the 96-well plate. Metabolites were extracted and derivatized for the quantitation of amino acids and biogenic amines. The extracts were finally diluted with MS running solvent before FIA and LC-MS/MS analysis. Three quality controls (QCs) composed of human plasma samples at three concentration levels: low (QC1), medium (QC2) and high (QC3), were used to evaluate the performance of the analytical assay. A seven-point serial dilution of calibrators was added to the 96-well plate of the kit to generate calibration curves for the quantification of amino acids and biogenic amines.

### Statistical analysis

Before statistical analysis, the raw metabolomics data were examined to exclude metabolites with more than 20% of concentration values below the lower limit of quantitation (LLOQ) or above the upper limit of quantitation (ULOQ).

Univariate analysis of metabolomics data was performed using the non-parametric Mann-Whitney-Wilcoxon test (hereinafter referred to as the Wilcoxon test) for quantitative variables and Fisher's exact test for qualitative variables. The Benjamini-Hochberg correction was used for comparing multiple metabolite concentrations to keep the false discovery rate (FDR) below 5%. Otherwise, the differences were considered significant at p ≤ 0.05.

Multivariate analysis was first performed with SIMCA-P v.14.0 (Umetrics, Umeå, Sweden), using principal component analysis (PCA) for the detection of sample grouping and outliers. We then applied the least absolute shrinkage and selection operator (LASSO) method on the mean centered-unit variance (MC-UV) scaled metabolites. With this method, a constraint or penalty is imposed on the coefficient of "classic" logistic regression to render the covariance matrix invertible, thus enabling computation of the estimated variance of the regression coefficients (Tibshirani, 1996. J R Stat Soc Series B Stat Methodol. 58(1):267-288). This method is considered suitable for the selection of variables since some coefficients are "shrunk" towards exactly zero under the constraint. For the LASSO procedure, data were randomly split a thousand times into a training set containing about two-thirds of all data, obtained from 23 POAG patients and 18 controls, and a test-set of data containing about one-third of all data, obtained from 12 POAG patients and 9 controls. A LASSO model was then constructed for each iteration. We thus obtained 1000 estimations for the coefficients of each metabolite, and for the area calculated under the receiving operator characteristic curve (AUROC), for the training and test-sets. The nullity of each coefficient was then tested using the Student's t-test, and a "volcano" plot (coefficients vs. p-values) was constructed to highlight the most important metabolites.

Since LASSO models using the whole set of metabolites might suffer from some degree of overfitting, we decided to integrate the most relevant metabolites shown by the volcano plot into a logistic regression (LR) model. Here again, the data were randomly split 1000 times into training (2/3 of the data) and test-sets (1/3 of the data), and the performance of the models was evaluated using the AUROC. Multivariate analysis was performed using the R packages glmnet (Friedman et al., 2010. J Stat Softw. 33(1):1-22), glm and ROCR packages (Sing et al., 2005. Bioinformatics. 21(20):3940-1) (http://www.Rproject. org).

### Results

After validation of the kit plate based on QC samples, 37 (19.7%) metabolites were excluded because the measurements were not considered sufficiently accurate. Statistical analysis was then carried out on the remaining 151 (80.3 %).

The unsupervised PCA scatter plot of metabolomics data showed no grouping according to the POAG or control groups, nor any strong outliers in the first principal plan (PC1 vs. PC2) **(****Figure 1****)**.

### Univariate analysis

After the Benjamini-Hochberg correction, univariate analysis showed significant differences between the POAG and control groups for 13 metabolites, with a global false discovery rate (FDR) of 0.045 **(Table 4).**

**Table 4. Univariate analysis showing the significant metabolites after type I error correction. Fold changes were calculated using median values for the glaucoma and control groups. Diacyl and acyl-alkyl phosphatidylcholines are represented as PC aa X:Y and PC ae X:Y, respectively, where X represents the sum of the lengths of two alkyl chains, and Y the sum of the unsaturation in both chains.**

| **Metabolite** | **Fold change (POAG/Control)** | ***p*-values** | **Corrected threshold for type I error** |
|---|---|---|---|
| **Spermine** | 0.97 | 0.00035946 | 0.00095347 |
| **Octadecadienoylcarnitine** | 0.82 | 0.00068322 | 0.00151273 |
| **Methionine Sulfoxide** | 1.31 | 0.00130063 | 0.00201902 |
| **Tyrosine** | 1.25 | 0.00130081 | 0.00249317 |
| **Spermidine** | 0.90 | 0.00170065 | 0.0029451 |
| **Octadecenoylcarnitine** | 0.76 | 0.00178562 | 0.00338041 |
| **Methionine** | 1.27 | 0.00232113 | 0.00380263 |
| **Propionylcarnitine** | 1.30 | 0.00360838 | 0.00421416 |
| **Hexose** | 1.09 | 0.00419376 | 0.00461671 |
| **Phosphatidylcholines diacyl 34:2** | 1.15 | 0.00471854 | 0.00501157 |
| **Phosphatidylcholines diacyl 36:4** | 1.15 | 0.00482577 | 0.00539975 |
| **Phosphatidylcholines diacyl 34:4** | 1.22 | 0.00515345 | 0.00578202 |
| **Phosphatidylcholine acyl-alkyl 40:1** | 1.18 | 0.00538276 | 0.00615902 |

### Multivariate analysis

Data were randomly split a thousand times into a training set (2/3 of the data) and a test-set (1/3 of the data), and a LASSO model was constructed for each iteration.

The estimated mean coefficients and the -log10(p-values), *i.e.,* the "volcano" plot for the thousand LASSO models obtained after splitting are shown in **Figure 2****.**

The metabolites with the most important coefficients obtained with the LASSO method, *i.e.,* octadecadienoylcarnitine (C18:2), spermidine, decanoylcarnitine (C10:1) and acyl-alkyl phosphatidylcholine 40:1 (PC ae 40:1), were used to build logistic regression (LR) models.

The mean AUROC values for the LASSO models predicting training and test-sets outcomes, *i.e.,* glaucoma patients vs. controls, were 98.64% (95% CI: 98.34-98.94%; p-value < 2.2e-16) and 81.16% (95% CI: 80.50-81.82; p-value < 2.2e-16), respectively **(****Figure 3A** and **B**).

The mean AUROC values for the logistic regression models predicting training and test-set outcomes, *i.e.,* glaucoma patients vs. controls, were 95.71% (95%CI: 95.60-95.83%; p-value < 2.2e-16) and 91.78% (95%CI: 91.41- 92.15; p-value< 2.2e-16), respectively (**Figure 3C** and **D**).

As expected, mean AUROCs (horizontal lines) were higher and AUROC values were less spread out for the training sets when compared to the test-sets for both the LASSO and the LR models. This result from the less accurate predictions of models applied to «unseen» data (*i.e.,* data which have not participated in the construction of the model, such as the data of the test-set) and the difference of size between the training and test-sets. Furthermore, ROC-AUC values for the test-sets were higher in the LR models compared to the LASSO models, indicating a better performance and less over-fitted LR models compared to LASSO models.

The difference between the training and test-set AUROC values for the LASSO (∼17.5%) and the logistic regression (∼4%) models, and the better performances of the latter on the test-sets (91.78 vs. 81.16%), are all indicative of a less over-fitted LR model compared to the LASSO models.

Of the total of 188 metabolites analyzed, 151 were accurately measured in the plasma of individuals with POAG, and 18 showed significant differences of concentration compared to age- and sex-matched controls. With arrows (↑) and (↓) respectively indicating significantly increased and decreased metabolite concentrations in POAG patients compared to controls, both univariate and multivariate analyses identified 7 of the 18 metabolites, i.e. spermidine (↓), spermine (↓), octadecadienoylcarnitine (C18:2) (↓), tyrosine (↑), methionine (↑), the group of hexoses (↑), and the phosphatidylcholine acyl-alkyl 40:1 (PC ae 40:1) (↑). Six metabolites were identified by univariate analysis alone, i.e. C18:1 octadecenoylcarnitine (↓), methionine sulfoxide (MetSO) (↑), C3 propionylcarnitine (↑), and the three phosphatidylcholines PC aa 34:2 (↑), PC aa 34:4 (↑), and PC aa 36:4 (↑); and 5 metabolites were identified by multivariate analysis alone, i.e. C4 butyrylcarnitine (↑), C10:1 decenoylcarnitine (↑), C12:1 dodecenoylcarnitine (↑), arginine (↑), and histamine (↓).

Metabolites found important according to univariate analysis, multivariate analysis, or both, are shown in **Figure 4****.**

### Conclusion

The analysis of the 18 metabolites contributing to the POAG metabolomic signature points toward three main, closely associated pathophysiological conditions:
i) faulty mitochondrial oxidation;
ii) senescence-like metabolic alteration; and
iii) deficiency of molecules known to be protective for the retinal ganglion cells.

The hexoses, measured as a single group of metabolites in our study, were found at a significantly increased concentration (+ 9%) in the plasma of individual with POAG compared to that of controls. Despite the frequent co-occurrence of diabetes and glaucoma, the increased concentration of hexoses could not be attributed either to diabetes or to obesity in the individual with POAG, who did not differ from the control population in terms of diagnoses of diabetes and antidiabetic medications. Moreover, the average BMI of individuals with POAG was significantly lower than that of controls. Our finding suggests a subtle systemic alteration of carbohydrate metabolism.

The POAG signature is also characterized by modified concentrations of six acylcarnitines. The concentrations of two short-chain acylcarnitines (C3 and C4) and two medium-chain acylcarnitines (C10:1 and C12:1) were increased, whereas the concentrations of two long-chain acylcarnitines (C18:2 and C18:1) were decreased. Our approach allowed a complete overview of the acylcarnitines that are involved in the mitochondrial mode of transport of fatty acids. An incomplete oxidation of fatty acids, leading to a reflux from mitochondria toward the blood of the smaller acylcarnitines may be at the origin of the increased acylcarnitine levels. In contrast the decreased concentration of the two-long chain acylcarnitines that we observed, is more likely to be involved in the senescence-like phenotype.

The increased concentration of the two main energetic suppliers represented by the sum of hexoses and acylcarnitines argues strongly in favor of a generalized mitochondrial defect of oxidation. The metabolomic signature we report concords with the notion of a subtle, generalized mitochondrial defect contributing to the pathogenesis of POAG.

Phosphatidylcholines are major phospholipids of cellular membranes. The accumulation of the four phosphatidylcholines found in our metabolomic signature has never before been reported in the blood of individuals with POAG.

Concerning the pool of discriminant amino acids and biogenic amines, a striking feature is the reduced concentration of spermidine and spermine in the POAG group of patients compared to controls. It is worth noting that methionine and arginine, which have increased concentrations in the metabolomic signature of POAG, are both precursors of the biosynthesis of spermidine and spermine, with methionine being a methyl radical donor.

In conclusion, our standardized targeted metabolomics study, conducted on the plasma of individuals with POAG, revealed a comprehensive metabolic signature combining the accumulation of mitochondrial energetic substrates (sum of hexoses, short- and medium-chain acylcarnitines) with senescence biomarkers (increased phosphatidylcholines and decreased spermine, spermidine and long-chain acylcarnitines).

## Claims

1. A metabolic signature comprising at least four, preferably at least twelve metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine,
wherein said metabolic signature is indicative of glaucoma in a subject.

2. The metabolic signature according to claim **1,** wherein:
- the amount, level or concentration of at least 1 metabolite, preferably at least 2 metabolites selected from the group comprising histamine, octadecadienoylcarnitine, octadecenoylcarnitine, spermidine and spermine is lower, and
- the amount, level or concentration of at least 1 metabolite, preferably at least 2 metabolites selected from the group comprising arginine, butyrylcarnitine, decenoylcarnitine, dodecenoylcarnitine, hexoses, methionine, methionine sulfoxide, PC aa 34:2, PC aa 34:4, PC aa 36:4, PC ae 40:1, propionylcarnitine and tyrosine is higher,
with respect to the amount, level or concentration of the same metabolite in a reference population.

3. The metabolic signature according to claim **2,** wherein the reference population comprises at least 10 subjects who are not affected and/or who have not been diagnosed with glaucoma.

4. A method for diagnosing glaucoma in a subject and/or for identifying a subject as being at risk for glaucoma, comprising the steps of:
a. measuring in a sample previously obtained from the subject the amount, level or concentration of at least four metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine, thereby obtaining a personalized metabolic signature,
b. comparing the personalized metabolic signature obtained in step b) with the metabolic signature according to any one of claims **1** to **3,** and
c. diagnosing the subject as being affected or as being at risk of being affected with glaucoma when the comparison of step c) reveals that the personalized metabolic signature provided in step b) is substantially similar to the metabolic signature according to any one of claims **1** to **3.**

5. A method for diagnosing glaucoma in a subject and/or for identifying a subject as being at risk for glaucoma, comprising the steps of:
a. measuring in a sample previously obtained from the subject the amount, level or concentration of at least four metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine,
b. comparing the amount, level or concentration of the at least four, preferably at least twelve, more preferably eighteen metabolites measured in step b) with the amount, level or concentration of the same at least four, preferably at least twelve, more preferably eighteen metabolites in a population of substantially healthy subjects, and
c. diagnosing the subject as being affected or as being at risk of being affected with glaucoma when
- the amount, level or concentration of at least 2 metabolites selected from the group comprising histamine, octadecadienoylcarnitine, octadecenoylcarnitine, spermidine and spermine measured in step b) is lower than the amount, level or concentration of the same metabolite in a reference population of substantially healthy subjects, and
- the amount, level or concentration of at least 2 metabolites selected from the group comprising arginine, butyrylcarnitine, decenoylcarnitine, dodecenoylcarnitine, hexoses, methionine, methionine sulfoxide, PC aa 34:2, PC aa 34:4, PC aa 36:4, PC ae 40:1, propionylcarnitine and tyrosine measured in step b) is higher than the amount, level or concentration of the same metabolite in a reference population of substantially healthy subjects.

6. The methods according to claim **4** or **5,** wherein step a) comprises measuring the amount, level or concentration of at least twelve metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine.

7. The methods according to any one of claims **4** to **6,** wherein step a) comprises measuring the amount, level or concentration of eighteen metabolites selected from the group comprising arginine, butyrylcarnitine (C4), decenoylcarnitine (C10:1), dodecenoylcarnitine (C12:1), hexoses, histamine, methionine, methionine sulfoxide, octadecenoylcarnitine (C18:1), octadecadienoylcarnitine (C18:2), PC aa 34:2, PC aa 34:4, PC aa 36:4, PC ae 40:1, propionylcarnitine (C3), spermidine, spermine and tyrosine.

8. The methods according to any one of claims **4** to **7,** wherein the amount, level or concentration of metabolites is measured by flow injection analysis coupled with tandem mass spectrometry (FIA-MS/MS) or by liquid chromatography coupled with mass spectrometry (LC-MS).

9. The methods according to any one of claims **4** to **8,** wherein glaucoma is open-angle glaucoma, preferably glaucoma is primary open-angle glaucoma.

10. The methods according to any one of claims **4** to **9,** wherein the sample is a blood sample, preferably a plasma sample.

11. A kit for implementing the method according to any one of claims **8** to **10,** wherein said kit comprises:
- means for determining the amount, level or concentration of at least one metabolite selected from the group comprising arginine, butyrylcarnitine, decenoylcarnitine, dodecenoylcarnitine, hexoses, histamine, methionine, methionine sulfoxide, octadecadienoylcarnitine, octadecenoylcarnitine, PC aa 34:2, PC aa 34:4, PC aa 36:4, PC ae 40:1, propionylcarnitine, spermidine, spermine and tyrosine,
- internal standards and/or control, and
- instructions for use.

12. The kit according to claim **11,** wherein internal standards and/or control are selected from:
- stable isotope-labelled arginine, stable isotope-labelled butyrylcarnitine, stable isotope-labelled decenoylcarnitine, stable isotope-labelled dodecenoylcarnitine, stable isotope-labelled hexoses, stable isotope-labelled histamine, stable isotope-labelled methionine, stable isotope-labelled methionine sulfoxide, stable isotope-labelled octadecadienoylcarnitine, stable isotope-labelled octadecenoylcarnitine, stable isotope-labelled PC aa 34:2, stable isotope-labelled PC aa 34:4, stable isotope-labelled PC aa 36:4, stable isotope-labelled PC ae 40:1, stable isotope-labelled propionylcarnitine, stable isotope-labelled spermidine, stable isotope-labelled spermine, stable isotope-labelled tyrosine, and/or
- stable isotope-labelled isobaric metabolites having the same molecular mass but different chemical formula as arginine, butyrylcarnitine, decenoylcarnitine, dodecenoylcarnitine, hexoses, histamine, methionine, methionine sulfoxide, octadecadienoylcarnitine, octadecenoylcarnitine, PC aa 34:2, PC aa 34:4, PC aa 36:4, PC ae 40:1, propionylcarnitine, spermidine, spermine or tyrosine.
